(19) Europäisches Patentamt
European Patent Office
Office européen des brevets

(11) Publication number: **0 516 154 A1**

# (12) EUROPEAN PATENT APPLICATION

(21) Application number: **92109109.6**

(51) Int. Cl.[5]: **C07H 17/04**, A61K 31/70

(22) Date of filing: **29.05.92**

(30) Priority: **30.05.91 US 707505**

(43) Date of publication of application:
**02.12.92 Bulletin 92/49**

(84) Designated Contracting States:
**AT BE CH DE DK ES FR GB GR IT LI LU MC NL PT SE**

(71) Applicant: **Bristol-Myers Squibb Company**
**345 Park Avenue**
**New York, N.Y. 10154(US)**

(72) Inventor: **Toda, Soichiro**
**3-591-5, Ohnari-cho**
**Ohmiya(JP)**
Inventor: **Yamashita,Haruhiro**
**3-3-295, Higashi-shisui**
**Shisui-machi, Inba-gun(JP)**
Inventor: **Naito, Takayuki**
**2657-45, Ohzenji Asao-ku**
**Kawasaki(JP)**
Inventor: **Nishiyama, Yuji**
**2-15-7-301, Kuramae**
**Taito-ku, Tokyo(JP)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Preparation of 6-0-acylelsamicin a derivatives.

(57) This invention relates to novel elsamicin A derivatives having 6-O-acyl substitutent, a process for producing said elsamicin A derivatives, antitumor compositions containing the same as the active ingredient, and a method for therapy using said compositions.

EP 0 516 154 A1

This invention relates to novel elsamicin A derivatives which have improved antitumor activity, lower toxicity and/or better pharmacokinetic properties, to their production, to compositions containing the same as the active ingredient, and a method for therapy using said compositions.

Elsamicin A is an antitumor antibiotic produced by cultivating an elsamicin A-producing strain of actinomycete designated strain J907-21 (ATCC 39417), or a mutant thereof. Elsamicin A exhibits antibacterial activity against aerobic gram-positive bacteria and anaerobic organisms. It also exerts activity against various murine tumor cells including leukemia P388, lymphoid leukemia L1210, and melanoma B16 in vitro and in vivo. Konishi, et al, Elsamicins, new antitumor antibiotics related to chartreusin. I. Production, isolation, characterization and antitumor activity, J. Antibiotics, 39: 784-791, (1986); U.S. Patent 4,518,589 to Konishi, et al, issued May 21, 1985.

The structure of elsamicin A (Formula I, below) has been determined and shown to be closely related to chartreusin (Formula II, below). Sugawara, et al, Elsamicins A and B, new antitumor antibiotics related to chartreusin. II. Structures of elsamicins A and B. J. Org. Chem., 52: 996-1001, (1987).

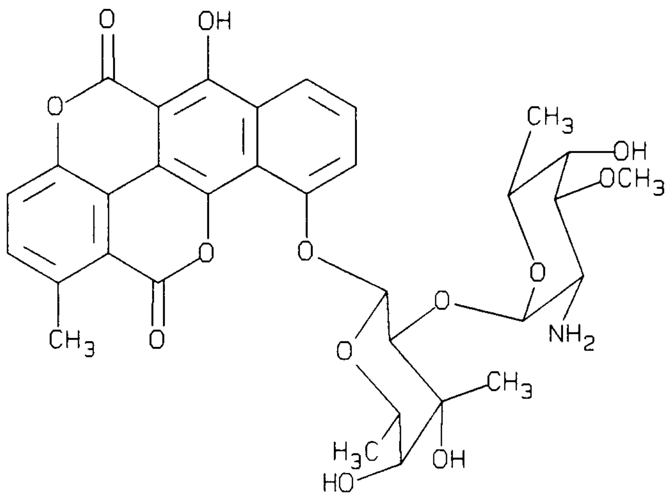

**Formula I**

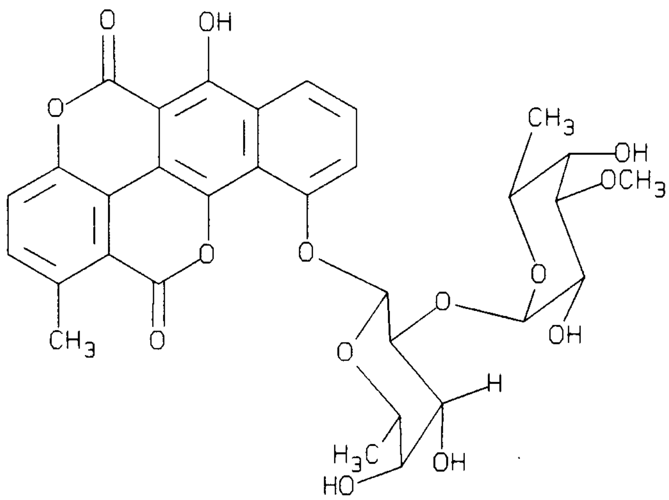

**Formula II**

Both elsamicin A and chartreusin have the same aglycone, chartarin, but the antibiotics differ in the disaccharide moiety. Leach, et al, Chartreusin, a new antibiotic produced by Streptomyces chartreusis, a new species, J. Am. Chem. Soc., 75: 4011-4012, (1953);
Beisler, J.A., Chartreusin, a glycosidic antitumor antibiotic for Streptomyces, In progress in Medicinal Chemistry, Ed., G.P. Ellis and G.B. West 19: pp. 247-268, Elsevier Biomedical Press, Amsterdam, (1982);

Simonitsch, et al: Über die Struktur des Chartreusins I, Helv. Chim. Acta, 47: 1459-1475, (1964); Eisenhuth, et al, Über die Struktur des Chartreusins II, Helv. Chim. Acta, 47, 1475-1484, (1964). Interconversion of both compounds by chemical process has never been reported.

In the course of chemical modification of elsamicin A, we found that acylation on the 6-position of elsamicin A gave new derivatives having improved antitumor activity, lower toxicity and/or better pharmacokinetic properties.

The present invention provides new derivatives of elsamicin A which exhibit improved antitumor activity, lower toxicity and/or better pharmacokinetic properties. More particularly the present invention provides the synthesis of 6-O-acylelsamicin A and their 3',4'-O-isopropylidene derivatives.

This invention further provides an antitumor composition comprising, as the active ingredient, at least one member selected from the group consisting of the elsamicin A derivatives of the present invention.

This invention further provides a method for therapy of cancer using the above antitumor composition.

Further provided is a process for producing the above-mentioned elsamicin A derivative.

U. S. Patent No. 4,518,589 to Konishi et al, discloses the production and isolation of the antitumor agent designated elsamicin A. (Formula I, above). The above-mentioned elsamicin A compound is the principal component of the fermentation of the elsamicin A-producing strain of actinomycete, designated strain J907-21 (ATCC 39417).

It has now been found according to the present invention that acylation on the 6-position of elsamicin A gave new derivatives having improved antitumor activity, lower toxicity and/or better pharmacokinetic properties.

The elsamicin A derivatives of the present invention have the general Formula III and IV below

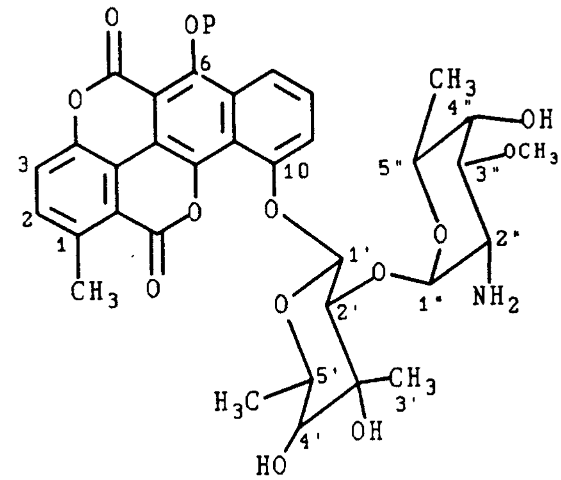

Formula III

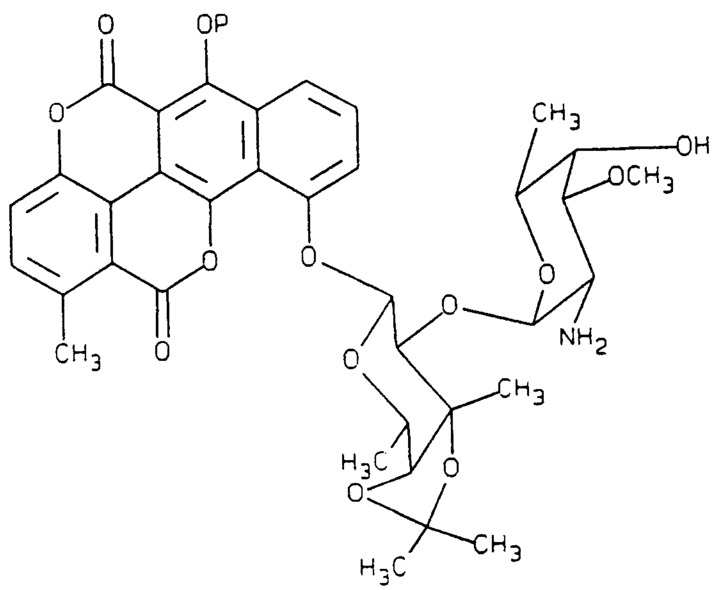

Formula IV

wherein P is COR; and R is $C_1$-$C_5$ alkyl which may be unsubstituted or substituted preferably with amino or acylamino.

The term "$C_1$-$C_5$ alkyl" as used herein and in the claims (unless the context indicates otherwise) means branched or straight chain hydrocarbon group having 1 to 5 carbon atoms such as methyl, ethyl, propyl, isopropyl, butyl, isobutyl, t-butyl, etc. Otherwise specified in the particular instance, the term "substituted or unsubstituted" as used herein and in the claims is intended to mean hydrocarbon group wherein an atom, element or group is regarded as having replaced a hydrogen atom, said substituted alkyl groups are preferably substituted with amino or acylamino. "Acyl" is preferably R'CO, wherein R' is hydrogen, $C_1$-$C_5$-alkyl or phenyl.

The elsamicin A derivatives of the present invention can be produced, for example, by the synthetic process diagramed in Scheme 1. As shown in Scheme 1, O-acylation of N-t-butoxycarbonyl elsamicin A (3) with appropriate carboxylic acids and dicyclohexylcarbodiimide (DCC) in the presence of a base such as pyridine, gave the intermediates 5 and subsequent deprotection gave the 6-O-acyl derivatives (7). The 6-0-acyl-3',4'-O-isopropylidene derivatives (8) were similarly prepared by acylation of N-t-butoxycarbonyl-3',4'-O-isopropylidene elsamicin A (4) followed by deblocking of the t-butoxycarbonyl group of the intermediates 6 with p-toluenesulfonic acid (TsOH) in acetone or trifluoroacetic acid (TFA). The isopropylidene group of

intermediates 6 was retained during the above deblocking process. Treatment of intermediates 6 with aqueous TFA simultaneously removed both the t-butoxycarbonyl and the isopropylidene groups to afford compound 7.

Table 1 indicates the compounds of the present application and their respective number.

Table 1

| Compound of the present invention and their respective number | |
|---|---|
| Compound No. | Name |
| 1 | Elsamicin A |
| 2 | Chartreusin |
| 3 | 2''-N-t-butoxycarbonylelsamicin A |
| 4 | 2''-N-t-butoxycarbonyl-3',4'-O-isopropylideneelsamicin A |
| 5a | 6-O-acetyl-2''-N-t-butoxycarbonylelsamicin A |
| 5c | 2''-N-t-butoxycarbonyle-6-O-(N-tirfluoroacetyl-$\beta$-alanyl)elsamicin A |
| 5d | 2''-N-t-butoxycarbonyl-6-O-(N-t-butoxycarbonyl-$\beta$-alanyl)elsamicin A |
| 5e | 2''-N-t-butoxycarbonyl-6-O-(N-t-butoxycarbonyl-L-alanyl)elsamicin A |
| 5f | 2''-N-t-butoxycarbonyl-6-O-(N-t-butoxycarbonyl-D-alanyl)elsamicin A |
| 6a | 6-O-acetyl-2''-N-t-butoxycarbonyl-3',4'-O-isopropylideneelsamicin A |
| 6b | 2''-N-t-butoxycarbonyl-6-O-n-hexanoyl-3',4'-O-isopropylideneelsamicin A |
| 6c | 2''-N-t-butoxycarbonyl-3',4'-O-isopropylidene-6-O-(N-trifluoroacetyl-$\beta$-alanyl)elsamicin A |
| 6d | 2''-N-t-butoxycarbonyl-6-O-(N-t-butoxycarbonyl-$\beta$-alanyl)-3',4'-O-isopropylideneelsamicin A |
| 6e | 2''-N-t-butoxycarbonyl-6-O(-N-t-butoxycarbonyl-L-alanyl)-3',4'-O-isopropylideneelsamicin A |
| 6f | 2''-N-t-butoxycarbonyl-6-O-(N-t-butoxycarbonyl-D-alanyl)-3',4'-O-isopropylideneelsamicin A |
| 7a | 6-O-acetylelsamicin A |
| 7b | 6-O-n-hexanoylelsamicin A |
| 7c | 6-O-(N-trifluoroacetyl-$\beta$-alanyl)elsamicin A |
| 7d | 6-O-$\beta$-alanylelsamicin A |
| 7e | 6-O-L-alanylelsamicin A |
| 7f | 6-O-D-alanylelsamicin A |
| 8a | 6-O-acetyl-3',4'-O-isopropylideneelsamicin A |
| 8b | 6-O-n-hexanoyl-3',4'-O-isopropylideneelsamicin A |
| 8c | 3',4'-O-isopropylidene-6-O-(N-trifluoroacetyl-$\beta$-alanyl)elsamicin A |
| 8d | 6-O-$\beta$-alanyl-3',4'-O-isopropylideneelsamicin A |
| 8e | 6-O-L-alanyl-3',4'-O-isopropylideneelsamicin A |
| 8f | 6-O-D-alanyl-3',4'-O-isopropylideneelsamicin A |

**Scheme 1**

**Synthetic Route of 6-O-Acyl Derivatives of Elsamicin A**

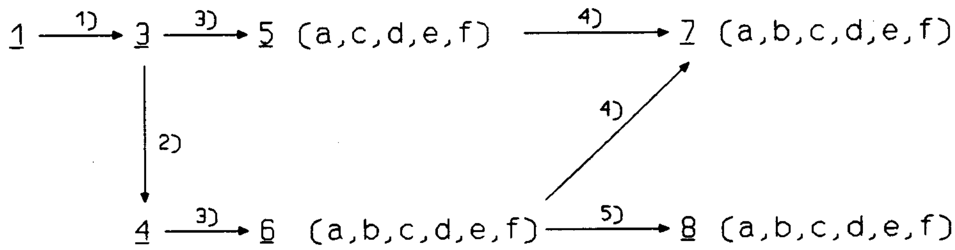


1) $(t\text{-}BOC)_2O/NEt_3$

2) $(CH_3O)_2C(CH_3)_2/TsOH$

3) R'COOH/Pyr/DCC

4) TFA

5) TFA or TsOH/acetone

**SCHEME 1 (cont'd)**

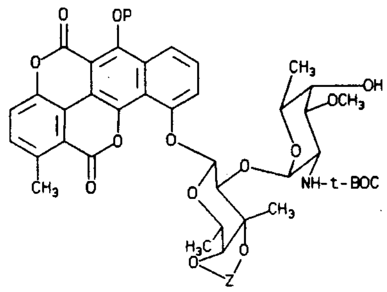

| Intermediate | P | -Z- |
|---|---|---|
| 3 | H | $(-H)_2$ |
| 4 | H | $C(CH_3)_2$ |
| 5 | COR' | $(-H)_2$ |
| 6 | COR' | $C(CH_3)_2$ |

R' a: $-CH_3$
b: $-(CH_2)_4-CH_3$
c: $-(CH_2)_2-NHCOCF_3$
d: $-(CH_2)_2-NH-t-BOC$
e: $-CHCH_3$ (L)
| NH-t-BOC
f: $-CHCH_3$ (D)
| NH-t-BOC

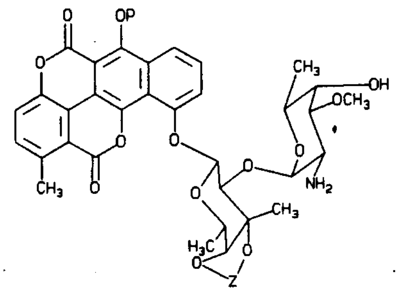

| Compound | P | -Z- |
|---|---|---|
| 7 | COR | $(-H)_2$ |
| 8 | COR | $C(CH_3)_2$ |

R a: $-CH_3$
b: $-(CH_2)_4-CH_3$
c: $-(CH_2)_2-NHCOCF_3$
d: $-(CH_2)_2-NH_2$
e: $-CHCH_3$ (L)
| $NH_2$
f: $-CHCH_3$ (D)
| $NH_2$

Antitumor activity of 6-0-acylelsamicin A derivatives

Twelve 6-0-acylelsamicin A derivatives were synthesized and comparatively tested with the parent compound for in vitro and in vivo antitumor activities.

For in vitro cytotoxicity experiment, murine melanoma B16-F10 cells were grown and maintained in Eagle's minimum essential medium (Nissui), which contains kanamycin (60 μg/ml), supplemented with heat-inactivated fetal calf serum (10%) and non-essential amino acids (0.6%) at 37°C under a humidified atmosphere in a 5% $CO_2$ incubator. Exponentially growing B16-F10 cells were harvested, counted and suspended in the culture medium at the concentration of $2.0x10^4$ cells/ml. The cell suspension (180 μl) was planted into wells of a 96-well microtiter plate and incubated for 24 hours. Test compounds (20 μl) were added to the wells and the plates were further incubated for 72 hours. The cytotoxic activity was colorimetrically determined at 540 nm after staining viable cells with neutral red solution. Among the twelve 6-O-acyl derivatives tested, seven derivatives showed approximately equipotent cytotoxicity to the parent compound against B16-F10 cells. As shown in Table 2, the seven derivatives are 6-O-acetylelsamicin A, (7a), 6-O-n-hexanoylelsamicin A, (7b), 6-O-(N-trifluoroacetyl-β-alanyl)elsamicin A, (7c), 6-O-β-alanylelsamicin A, (7d), 6-O-n-hexanoyl-3',4'-O-isopropylideneelsamicin A, (8b), 3'4'-0-isopropylidene-6-O-(N-trifluoroacetyl-β-alanyl)elsamicin A, (8c) and 6-O-β-alanyl-3',4'-O-isopropylideneelsamicin A, (8d). The other five derivatives were less active.

In vivo antitumor activity of the above twelve 6-O-acyl derivatives was tested in the lymphocytic leukemia P388 and B16 melanoma systems. Female $CDF_1$ (for P388) and male $BDF_1$ (for B16) mice were inoculated by ip injection at $10^6$ P388 cells and 0.5 ml of a 10% B16 brei per mouse, respectively (day 0). Test compounds were intraperitoneally administered to the mice once daily on days 1 to 3 (Q1Dx3) in the P388 system or once a day on days 1, 5 and 9 (Q4Dx3) in the B16 system and animals were observed for 50 days. The percent increase of median survival time (MST) of treated animals over that of untreated control animals was determined and reported as T/C %. Compounds showing T/C % values of 125 or greater were considered to have significant antitumor activity. As shown in Table 2, several derivatives gave promising antitumor activity against P388 leukemia and the minimum effective dose (MED) values of the seven derivatives which showed equipotent cytotoxicity to the parent compound were also the same as that of elsamicin A, (1). In particular, 6-O-acetylelsamicin A, (7a), 6-O-(N-trifluoroacetyl-β-alanyl)elsamicin A, (7c), and 6-O-β-alanylelsamicin A (7d) exhibited superior antitumor activity to elsamicin A, (1) in terms of therapeutic index (toxic dose/MED). Among the five less cytotoxic derivatives, antitumor activity of four derivatives, except for 6-O-n-hexanoyl-3',4'-O-isopropylideneelsamicin A (8b), was not significantly different from that of the parent compound. 6-O-Acetylelsamicin A (7a), 6-O-n-hexanoylelsamicin A (7b), 6-O-β-alanylelsamicin A (7d), 6-O-n-hexanoyl-3',4'-O-isopropylideneelsamicin A (8b) and 6-O-D-alanyl-3'4'-O-isopropylideneelsamicin A, (8f) were as active as elsamicin A (1) in the B16 system (Table 3). Interestingly, while the 6-O-acetyl-3',4'-isopropylideneelsamicin A, (8a) and 6-0-L-alanyl-3',4'-O-isopropylideneelsamicin A, (8e) derivatives were less active than the parent compound, these remarkably prolonged the life-span in the B16-bearing mice.

Table 2 In vitro cytotoxicity against B16 melanoma and in vivo antitumor activity against P388 leukemia in mice.

| | | Cytotoxicity | T/C % of MST*1 | | | | | |
|---|---|---|---|---|---|---|---|---|
| Compound | -COR | $IC_{50}$ (μg/ml) | 20*2 | 10 | 3 | 1 | 0.3 | 0.1 |
| 7a | $COCH_3$ | 0.06 | 247 | 185 | 160 | 145 | 130 | 120 |
| 7b | $CO(CH_2)_4CH_3$ | 0.04 | | 170 | 165 | 140 | 130 | 110 |
| 7c | $CO(CH_2)_2NHCOCF_3$ | 0.03 | 211 | 170 | 150 | 130 | 125 | 110 |
| 7d | $CO(CH_2)_2NH_2$ | 0.03 | 255 | 160 | 170 | 150 | 135 | 115 |
| 7e | $COCH(NH_2)CH_3$(L) | 0.3 | 186 | 176 | 157 | 124 | 114 | 105 |
| 7f | $COCH(NH_2)CH_3$(D) | 0.3 | 182 | 200 | 152 | 133 | 124 | 119 |
| 8a | $COCH_3$ | 0.09 | 255 | 224 | 171 | 152 | 124 | 114 |
| 8b | $CO(CH_2)_4CH_3$ | 0.05 | | 225 | 210 | 150 | 130 | 115 |
| 8c | $CO(CH_2)_2NHCOCF_3$ | 0.04 | | 260 | 230 | 160 | 135 | 110 |
| 8d | $CO(CH_2)_2NH_2$ | 0.04 | | 230 | 175 | 160 | 130 | 110 |
| 8e | $COCH(NH_2)CH_3$(L) | 0.16 | | 248 | 210 | 152 | 124 | 124 |
| 8f | $COCH(NH_2)CH_3$(D) | 0.3 | | 200 | 171 | 157 | 135 | 124 |
| 1 | | 0.04 | Tox | 201 | 171 | 152 | 136 | 123 |

*1 Median Survival time in days

*2 Dose in mg/kg/day, Q1Dx3 ip

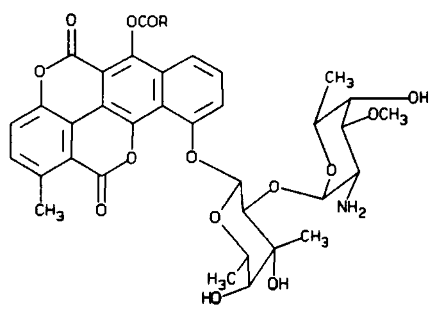

7

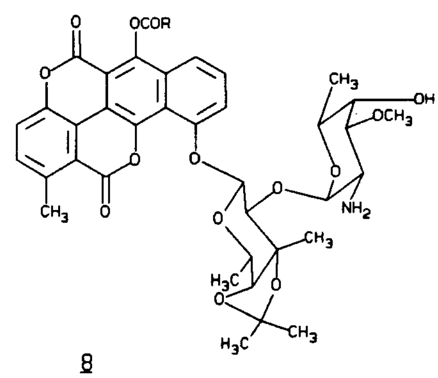

8

Table 3

| In vivo antitumor activity against B16 melanoma in mice | | | | | |
|---|---|---|---|---|---|
| Compound | T/C % of MST*1 | | | | |
| | 10*2 | 3 | 1 | 0.3 | 0.1 |
| 7a | 229 | 182 | 154 | 136 | 121 |
| 7b | 257 | 186 | 154 | 136 | 114 |
| 7c | 184 | 158 | 134 | 116 | 113 |
| 7d | 315 | 215 | 173 | 146 | 123 |
| 7f | 210 | 173 | 137 | 110 | |
| 8a | 287 (1/4)*3 | 280 (1/4) | 170 | 123 | |
| 8b | 254 | 182 | 164 | 132 | 111 |
| 8c | ≧250 (2/4) | 179 | 121 | 115 | |
| 8d | 296 | 211 | 139 | 121 | 118 |
| 8e | ≧333 (3/4) | 180 | 153 | 123 | |
| 8f | 240 | 180 | 153 | 140 | 107 |
| 1 | 287 (6/24) | 211 (3/24) | 179 | 142 | 122 |

*1 Median survival time in days
*2 Dose in mg/kg/day, Q4Dx3 ip
*3 No. of survivors/tested on day 50

The present invention includes within its scope a process for producing the elsamicin A derivatives of the present invention.

Another aspect of the invention, there are provided pharmaceutical compositions which comprise an effective tumor-inhibiting amount of the compound of Formula III or IV, in combination with an inert pharmaceutically acceptable carrier or diluent.

According to another aspect of the invention provides a method for therapeutically treating an animal, preferably mammalian, host affected by a tumor which comprises administering to such host an effective tumor-inhibiting dose of the antibiotic of the compound of Formula III or IV.

Examples of suitable compositions include solid compositions for oral administration such as tablets, capsules, pills, powders and granules, liquid compositions for oral administration such as solutions, suspensions, syrups and elixirs and preparations for parenteral administration such as sterile solutions, suspensions or emulsions. They may also be manufactured in the form of sterile solid compositions which can be dissolved in sterile water, physiological saline or some other sterile injectable medium immediately before use.

It will be appreciated that the actual preferred dosages of the elsamicin A derivative of the present invention will vary according to the particular compound being used, the particular composition formulated, the mode of application and the particular situs, host and disease being treated. Many factors that modify the action of the drug will be taken into account by those skilled in the art, e.g. age, body weight, sex, diet, time of administration, rate of excretion, condition of the host, drug combinations, reaction sensitivities and severity of the disease. Administration can be carried out continuously or periodically within the maximum tolerated dose. Optimal application rates for a given set of conditions can be readily ascertained by those skilled in the art using conventional dosage determination tests.

The present invention is illustrated by the following examples which are not intended to be construed as limiting the scope of the invention.

Specific synthesis examples of the intermediates 3 - 6(f) are explained below, from which intermediates the compounds of the present invention 7a - 7f and 8a - 8f are synthesized by the above process.

EXAMPLE 1

Synthesis of 2''-N-t-Butoxycarbonylelsamicin A (3)

A mixture of elsamicin A (1), (653 mg, 1 mmole), di-t-butyl dicarbonate (348 mg, 1.6 mmoles) and triethylamine (0.14 ml, 1 mmole) in dioxane (10 ml) was stirred at room temperature overnight. The reaction mixture was evaporated in vacuo to give a semi-crystalline residue, which was recrystallized from

$CH_2Cl_2$/ether to obtain 768 mg (100%) of intermediate 3 as yellow crystalline powder.

MP 183-184°C. IR $\nu_{max}$ (KBr) $cm^{-1}$ 3410, 1700, 1505, 1370, 1255, 1120, 1065, 875, 780. UV $\lambda_{max}$ (MeOH) nm ($\epsilon$) 236 (38300), 266 (37800), 333 (6180), 380 (8770), 400 (14500), 423 (15900). $^1H$ NMR ($CDCl_3$) $\delta$ 0.73 (9H, br.s.), 1.31 (3H, d, J=7.0 Hz), 1.36 (3H, s), 1.39 (3H, d, J=6 Hz), 2.68 (3H, s), 3.35 (3H, s), 5.37 (1H, d, J=8.0 Hz), 4.66 (1H, d, J=4 Hz), 8.18 (1H, dd, J=8.0 & 1.5 Hz), 11.59 (1H, s).

| Anal. Calcd. for $C_{38}H_{43}NO_{15}\cdot H_2O$: | | | |
|---|---|---|---|
| | C 59.14, | H 5.88, | N 1.81. |
| Found: | C 59.12, | H 6.06, | N 2.27. |

EXAMPLE 2

Synthesis of 2''-N-t-Butoxycarbonyl-3',4'-0-isopronylideneelsamicin A (4)

To a solution of intermediate 3 (200 mg) and 2,2-dimethoxypropane (1.2 ml) in dry $CH_2Cl_2$ (4 ml) was added TsOH (5 mg) and the mixture was kept at room temperature overnight. Saturated aqueous $NaHCO_3$ was added to the reaction mixture and the organic layer was separated, dried over $MgSO_4$ and evaporated in vacuo to give 190 mg (90%) of intermediate 4 as a yellow solid.

MP 168-169°C. IR $\nu_{max}$ (KBr) $cm^{-1}$ 3420, 1740, 1690, 1505, 1375, 1250, 1065, 780. UV $\lambda_{max}$ (MeOH) nm ($\epsilon$) 236 (40600), 266 (39100), 333 (6370), 380 (8850), 399 (14200), 422 (15500). $^1H$ NMR ($CDCl_3$) $\delta$ 1.34 (3H, d, J=6 Hz), 1.37 (3H, d, J=7 Hz), 1.37 (3H, s), 1.42 (3H, s), 1.68 (3H, s), 2.87 (3H, s), 3.35 (3H, s), 5.23 (1H, d, J=8 Hz), 5.80 (1H, d, J=4 Hz), 8.33 (1H, dd, J=8 & 1.5 Hz), 1.63 (1H, br.).

| Anal. Calcd. for $C_{41}H_{47}NO_{15}\cdot H_20$: | | | |
|---|---|---|---|
| | C 60.66, | H 6.08, | N 1.73. |
| Found: | C 60.92, | H 6.03, | N 2.00. |

EXAMPLE 3

Synthesis of 6-0-Acetyl-2''-N-t-butoxycarbonylelsamicin A (5a)

A solution of 2''-N-t-butoxycarbonylelsamicin A (3) (79 mg, 0.1 mmole), acetic acid (12 mg, 0.2 mmole), DCC (33 mg, 0.16 mmole) and dry pyridine (0.5 ml) in ethyl acetate (1 ml) was kept at room temperature overnight. The mixture was poured into diluted aqueous HCl (30 ml) and extracted with EtOAc and the extract was washed subsequently with diluted aqueous HCl, $H_2O$ and saturated aqueous NaCl and evaporated in vacuo. The residue was purified by silica gel chromatography using MeOH/$CHCl_3$ solution as an eluant to give 66 mg (79%) of intermediate 5a as yellow amorphous powder.

MP 184-186°C. IR $\nu_{max}$ (KBr) $cm^{-1}$ 1775, 1735, 1365, 1250, 1165, 1065, 1035, 770. UV $\lambda_{max}$ (MeOH) nm ($\epsilon$) 236 (36500), 267 (41200), 311 (6190), 326 (7380), 366 (10800), 389 (12300), 410 (13000). $^1H$ NMR ($CDCl_3$) $\delta$ 0.77 (9H, s), 1.27 (3H, d, J=6 Hz), 1.37 (3H, s), 1.42 (3H, d, J=6 Hz), 2.59 (3H, s), 2.80 (3H, s), 3.38 (3H, s), 5.39 (1H, d, J=8 Hz), 5.60 (1H, d, J=4 Hz), 7.93 (1H, dd, J=8.0 & 1.5 Hz).

| Anal. Calcd. for $C_{40}H_{45}NO_{16}\cdot H_20$: | | | |
|---|---|---|---|
| | C 59.03, | H 5.82, | N 1.72. |
| Found: | C 59.33, | H 5.76, | N 1.68. |

EXAMPLE 4

Synthesis Of Intermediates Nos. 5c - 5f

Intermediates 5c - 5f were prepared by the similar method to that of the preparation of intermediate 5a

from intermediate 3. Acylating acids and isolation yields are given.

(a) Synthesis of 2''-N-t-Butoxycarbonyl-6-0-(N-trifluoroacetyl-β-alanyl)elsamicin A (5c)

N-Trifluoroacetyl-β-alanine (82%). MP 170-173° C. IR $\nu_{max}$ (KBr) $cm^{-1}$ 1740(sh), 1720, 1370, 1250, 1160, 1070, 775. UV $\lambda_{max}$ (MeOH) nm (ε) 236 (36600), 267 (40800), 311 (6280), 325 (7460), 366 (9210), 390 (12100), 411 (12600). $^1H$ NMR ($CDCl_3$) δ 0.73 (9H, s), 1.33 (3H, d, J=7 Hz), 1.38 (3H, s), 1.40 (3H, d, J=6 Hz), 2.78 (3H, s), 3.38 (3H, s), 5.43 (1H, s, J=8 Hz), 5.69 (1H, d, J=4 Hz).

| Anal. Calcd. for $C_{43}H_{47}N_2O_{17}F_3 \bullet H_20$: | | | |
|---|---|---|---|
| | C 55.01, | H 5.26, | N 2.98. |
| Found: | C 54.95, | H 5.16, | N 3.19. |

(b) Synthesis of 2''-N-t-Butoxycarbonyl-6-0-(N-t-butoxycarbonyl-β-alanyl)elsamicin A (5d)

N-t-Butoxycarbonyl-β-alanine (71%). MP 171-173° C. IR $\nu_{max}$ (KBr) $cm^{-1}$ 3420, 1740, 1710, 1610, 1580, 1370, 1255, 1165. UV $\lambda_{max}$ (MeOH) nm (ε) 236 (36700), 268 (41800), 311 (6410), 326 (7630), 365 (9400), 390 (12700), 410 (13100). $^1H$ NMR ($CDCl_3$) δ 0.79 (9H, s), 1.1-1.75 (22H, m), 2.84 (3H, s), 3.40 (3H, s), 5.40 (1H, d, J=8 Hz), 5.63 (1H, d, J=4 Hz).

| Anal. Calcd. for $C_{46}H_{56}N_2O_{18} \bullet H_20$: | | | |
|---|---|---|---|
| | C 58.59, | H 6.20, | N 2.97. |
| Found: | C 58.44, | H 5.90, | N 2.78. |

(c) Synthesis of 2''-N-t-Butoxycarbonyl-6-0-(N-t-butoxycarbonyl-L-alanyl)elsamicin A (5e)

N-t-Butoxycarbonyl-L-alanine (80%). MP 180-182° C. IR $\nu_{max}$ (KBr) $cm^{-1}$ 3420, 1740, 1610, 1500, 1370, 1250, 1160, 1070. UV $\lambda_{max}$ (MeOH) nm (ε) 237 (35700), 267 (40000), 312 (6250), 326 (7380), 367 (9200), 389 (12100), 410 (12800). $^1H$ NMR ($CDCl_3$) δ 0.75 (9H, s), 1.27 (3H, d, J=6 Hz), 1.37 (3H, s), 1.40 (3H, d, J=6 Hz), 1.50 (9H, s), 1.77 (3H, d, J=7 Hz), 2.81 (3H, s), 3.38 (3H, s), 5.39 (1H, d, J=8 Hz), 5.63 (1H, d, J=4 Hz).

| Anal. Calcd. for $C_{46}H_{56}N_2O_{18} \bullet H_20$: | | | |
|---|---|---|---|
| | C 58.59, | H 6.20, | N 2.97. |
| Found: | C 58.70, | H 6.12, | N 2.72. |

(d) Synthesis of 2''-N-t-Butoxycarbonyl-6-0-(N-t-butoxycarbonyl-D-alanyl)elsamicin A (5f)

N-t-Butoxycarbonyl-D-alanine (81%). MP 180-182° C. IR $\nu_{max}$ (KBr) $cm^{-1}$ 3420, 1740, 1610, 1450, 1370, 1250, 1165, 1070. UV $\lambda_{max}$ (MeOH) nm (ε) 236 (35500), 267 (39800), 312 (6260), 326 (7420), 366 (9210), 389 (12200), 410 (12900). $^1H$ NMR ($CDCl_3$) δ 0.8 (9H, s), 1.26 (3H, d, J=6 Hz), 1.38 (3H, s), 1.40 (3H, d, J=6 Hz), 1.49 (9H, s), 1.78 (3H, d, J=7 Hz), 2.81 (3H, s), 3.39 (3H, s), 5.38 (1H, d, J=8 Hz), 5.60 (1H, d, J=4 Hz).

| Anal. Calcd. for $C_{46}H_{56}H_2O_{18} \bullet 3/2H_20$: | | | |
|---|---|---|---|
| | C 59.15, | H 6.15, | N 3.00. |
| Found: | C 59.36, | H 6.18, | N 2.71. |

EXAMPLE 5

Synthesis of 6-0-Acetyl-2''-N-t-butoxycarbonyl-3',4'-0-isopronylideneelsamicin A (6a)

A solution of intermediate 4 (123 mg), acetic acid (20 mg), DCC (33 mg) and pyridine (0.75 ml) in EtOAc (2 ml) was kept at room temperature overnight. The reaction mixture was filtrated and evaporated in vacuo. The residue was purified by column chromatography on silica gel (4.5 x 16 mm) using MeOH in $CHCl_3$ as an eluant to give 128 mg (99%) of the desired compound.

MP 188-190 °C. IR $\nu_{max}$ (KBr) $cm^{-1}$ 3450, 1780, 1745, 1500, 1370, 1255, 1170, 1070. UV $\lambda_{max}$ (MeOH) nm ($\epsilon$) 236 (36500), 267 (41900), 310 (6310), 324 (7620), 364 (9280), 389 (12000), 409 (12300). $^1H$ NMR ($CDCl_3$) $\delta$ 0.67 (3H, s), 1.3-1.5 (12H, m), 1.70 (3H, s), 2.60 (3H, s), 2.90 (3H, s), 3.39 (3H, s), 5.25 (1H, d, J=8 Hz), 5.82 (1H, d, J=4 Hz).

| Anal. Calcd. for $C_{43}H_{49}NO_{16}\cdot 1/2H_2O$: | | | |
|---|---|---|---|
| | C 61.13, | H 5.97, | N 1.66. |
| Found: | C 60.86, | H 5.84, | N 1.73. |

EXAMPLE 6

Synthesis of Intermediates Nos. 6b - 6f

Intermediates 6b - 6f were prepared by the similar method to that described in the preparation of intermediate 6a from intermediate 4. Acylating acids, reaction time and isolation yields are given.

(a) Synthesis of 2''-N-t-Butoxycarbonyl-6-0-n-hexanoyl-3',4'-0-isopronylideneelsamicin A (6b)

Caproic acid, overnight, (76%). MP 184-186 °C. IR $\nu_{max}$ (KBr) $cm^{-1}$ 1740, 1495, 1370, 1250, 1215, 1170, 1125, 1070, 780. UV $\lambda_{max}$ (MeOH) nm ($\epsilon$) 236 (38300), 267 (43000), 311 (6380), 325 (7910), 365 (9550), 389 (12500), 409 (13000). $^1H$ NMR ($CDCl_3$) $\delta$ 0.67 (9H, s), 1.34 (3H, d, J=6 Hz), 1.41 (3H, d, J=6 Hz), 1.42 (3H, s), 1.46 (3H, s), 1.71 (3H, s), 2.92 (3H, s), 3.40 (3H, s), 5.25 (1H, d, J=8 Hz), 5.77 (1H, d, J=4 Hz).

| Anal. Calcd. for $C_{47}H_{57}NO_{16}\cdot 1/2H_2O$: | | | |
|---|---|---|---|
| | C 62.66, | H 6.49, | N 1.55. |
| Found: | C 62.81, | H 6.37, | N 1.58. |

(b) Synthesis of 2''-N-t-Butoxycarbonyl-3',4'-0-isopropylidene-6-0-(N-trifluoroacetyl-$\beta$-alanyl)elsamicin A - (6c)

N-Trifluoroacetyl-$\beta$-alanine, 24 hours, (93%). MP 169-170 °C. IR $\nu_{max}$ (KBr) $cm^{-1}$ 1740, 1720, 1500, 1370, 1255, 1215, 1165, 1120, 1070, 780. UV $\lambda_{max}$ (MeOH) nm ($\epsilon$) 236 (36500), 267 (41500), 310 (6500), 325 (7720), 365 (9390), 390 (12100), 410 (12200). $^1H$ NMR ($CDCl_3$) $\delta$ 0.66 (9H, s), 1.36 (3H, d, J=6 Hz), 1.38 (3H, d, J=6 Hz), 1.42 (3H, s), 1.46 (3H, s), 1.71 (3H, s), 2.92 (3H, s), 3.38 (3H, s), 5.24 (1H, d, J=8 Hz), 5.84 (1H, d, J=4 Hz).

| Anal. Calcd. for $C_{46}H_{51}N_2O_{17}F_3\cdot H_2O$: | | | |
|---|---|---|---|
| | C 56.44, | H 5.46, | N 2.86. |
| Found: | C 56.50, | H 5.23, | N 2.74. |

(c) Synthesis of 2''-N-t-Butoxycarbonyl-6-0-(N-t-butoxycarbonyl-$\beta$-alanyl)-3',4'-0-isopropylideneelsamicin A - (6d)

N-t-Butoxycarbonyl-β-alanine, 67 hours, (81%). MP 171-173°C. IR $\nu_{max}$ (KBr) $cm^{-1}$ 3400, 1740, 1710, 1610. UV $\lambda_{max}$ (MeOH) nm (ε) 236 (35200), 267 (40200), 310 (6240), 325 (7540), 364 (9020), 389 (11800), 409 (11900). $^1H$ NMR ($CDCl_3$) δ 0.67 (9H, s), 1.40 (18H, m), 1.62 (3H, s), 1.71 (3H, s), 2.82 (3H, s), 3.10 (2H, t, J=6 Hz), 3.40 (3H, s), 5.24 (1H, d, J=8 Hz), 5.80 (1H, d, J=4 Hz).

| Anal. Calcd. for $C_{49}H_{60}N_2O_{18}$: | | | |
|---|---|---|---|
| | C 60.99, | H 6.27, | N 2.90. |
| Found: | C 60.79, | H 6.12, | N 2.83. |

(d) Synthesis of 2''-N-t-Butoxycarbonyl-6-0-(N-t-butoxycarbonyl-L-alanyl)-3',4'-0-isopropylideneelsamicin A - (6e)

N-t-Butoxycarbonyl-L-alanine, 93 hours, (86%). MP 174-176°C. IR $\nu_{max}$ (KBr) $cm^{-1}$ 3420, 1745, 1710, 1500, 1370, 1250, 1070. UV $\lambda_{max}$ (MeOH) nm (ε) 236 (35800), 267 (40500), 311 (6290), 324 (7510), 364 (9160), 389 (12000), 410 (12300). $^1H$ NMR ($CDCl_3$) δ 0.67 (9H, s), 1.40 (21H, m), 1.70 (3H, s), 1.77 (3H, d, J=7 Hz), 2.92 (3H, s), 3.39 (3H, s), 5.24 (1H, d, J=8 Hz), 5.81 (1H, d, J=4 Hz).

| Anal. Calcd. for $C_{46}H_{60}N_2O_5S_{18}$•1/2$H_2O$: | | | |
|---|---|---|---|
| | C 60.42, | H 6.31, | N 2.88. |
| Found: | C 60.21, | H 6.32, | N 2.74. |

(e) Synthesis of 2''-N-t-Butoxycarbonyl-6-0-(N-t-butoxycarbonyl-D-alanyl)-3',4'-0-isopropylideneelsamicin A - (6f)

N-t-Butoxycarbonyl-D-alanine, 46 hours, (87%). MP 174-176°C. IR $\nu_{max}$ (KBr) $cm^{-1}$ 3420, 1745, 1710, 1450, 1370, 1250, 1070. UV $\lambda_{max}$ (MeOH) nm (ε) 236 (34400), 267 (39300), 311 (6180), 324 (7410), 364 (8960), 389 (11600), 409 (11900). $^1H$ NMR ($CDCl_3$) δ 0.65 (9H, s), 1.4-1.76 (32H, m), 2.90 (3H, s), 3.36 (3H, s), 4.85 (1H, q, J=7.5 Hz), 5.23 (1H, d, J=8 Hz), 5.79 (1H, d, J=4 Hz).

| Anal. Calcd. for $C_{49}H_{60}N_2O_{18}$•3/2$H_2O$: | | | |
|---|---|---|---|
| | C 60.42, | H 6.31, | N 2.88. |
| Found: | C 60.19, | H 6.33, | N 2.74. |

EXAMPLE 7

Synthesis of 6-0-Acetylelsamicin A (7a)

A mixture of intermediate 5a (55 mg) and trifluoroacetic acid (0.5 ml) was stirred for 5 minutes at room temperature. The mixture was evaporated under reduced pressure to give a sticky solid, which was triturated with ether to give 60 mg of compound 7a as yellow powder.

MP 189-191°C. IR $\nu_{max}$ (KBr) $cm^{-1}$ 1780, 1735, 1680, 1370, 1260, 1210, 1140, 1075, 780. UV $\lambda_{max}$ - (MeOH) nm (ε) 237 (35500), 267 (40100), 309 (6110), 324 (7350), 365 (8770), 388 (11600), 408 (11900). $^1H$ NMR ($CDCl_3$ + $CD_3OD$) δ 1.19 (3H, d, J=7.6 Hz), 1.37 (3H, d, J=6 Hz), 1.42 (3H, s), 2.52 (3H, s), 2.71 (3H, s), 3.45 (3H, s), 5.74 (1H, d, J=8 Hz), 5.83 (1H, d, J=4 Hz).

| Anal. Calcd. for $C_{35}H_{37}NO_{14}$•$CF_3COOH$•3$H_2O$: | | | |
|---|---|---|---|
| | C 51.45, | H 5.13, | N 1.62. |
| Found: | C 51.42, | H 4.32, | N 1.47. |

MS: (SIMS) M/Z 696 $(M+H)^+$, 377, 334, 160.

EXAMPLE 8

Synthesis of 6-0-n-Hexanoylelsamicin A (7b)

A mixture of intermediate 6b (60 mg) and 90% aqueous trifluoroacetic acid (0.5 ml) was kept at room temperature for 20 minutes. The mixture was concentrated and triturated to give yellow powder, which was purified by column chromatography on silica gel using MeOH in $CHCl_3$ (2% - 10%) as eluant to afford 29 mg (57%) of the desired product.

MP 184-187 °C. IR $\nu_{max}$ (KBr) $cm^{-1}$ 1735, 1675, 1255, 1205, 1135, 1070, 805, 780, 720. UV $\nu_{max}$ - (MeOH) nm ($\epsilon$) 237 (26800), 267 (29700), 310 (4590), 325 (5590), 365 (6660), 388 (8760), 409 (9110). $^1H$ NMR ($CDCl_3$) $\delta$ 0.99 (3H, t, J=7 Hz), 1.23 (3H, d, J=6 Hz), 1.37 (3H, d, J=6 Hz) 1.43 (3H, s), 2.85 (3H, s), 3.43 (3H, s), 5.63 (1H, d, J=8 Hz), 5.76 (1H, d, J=4 Hz).

MS (SIMS): M/Z 752 $(M+H)^+$, 654, 433, 334, 320, 100, 99, 86.

EXAMPLE 9

Synthesis of 6-0-(N-Trifluoroacetyl-β-alanyl)elsamicin A (7c)

A mixture of intermediate 5c (110 mg) and trifluoroacetic acid (0.5 ml) was sonicated until the mixture became homogeneous and then the mixture was evaporated under reduced pressure. The residue was dissolved in a small amount of aqueous t-BuOH and lyophilized to give 104 mg (93%) of the title compound.

MP 169-171 °C. IR $\nu_{max}$ (KBr) $cm^{-1}$ 1740(sh), 1720, 1365, 1255, 1200, 1170, 1070, 800, 755, 720. UV $\lambda_{max}$ (MeOH) nm ($\epsilon$) 237 (36100), 267 (40200), 310 (6230), 324 (7490), 365 (8900), 388 (11700), 408 (12000). $^1H$ NMR ($CDCl_3$ + $CD_3OD$) $\delta$ 1.19 (3H, d, J=7 Hz), 1.28 (3H, d, J=7 Hz), 1.43 (3H, s), 2.72 (3H, s), 3.46 (3H, s), 5.73 (1H, d, J=8 Hz), 5.87 (1H, d, J=4 Hz).

| Anal. Calcd. for $C_{38}H_{39}N_2O_{15}F_3 \cdot CF_3COOH \cdot 5/2H_2O$: | | | |
|---|---|---|---|
| | C 49.03, | H 4.63, | N 2.86. |
| Found: | C 48.91, | H 4.08, | N 3.12. |

EXAMPLE 10

Synthesis Of Compounds Nos. 7d -7f

Compounds 7d - 7f were prepared from intermediates numbers 5d - 5f, respectively by the similar method to that described in the preparation of compound 7c from intermediate 5c. Isolation yields are given.

(a) Synthesis of 6-0-β-Alanylelsamicin A (7d)

94 mg (91%), MP 177-179 °C (dec.). IR $\nu_{max}$ (KBr) $cm^{-1}$ 3400, 1740, 1730, 1670, 1255, 1170, 1070. UV $\lambda_{max}$ (MeOH) nm ($\epsilon$) 236 (34700), 268 (38800), 309 (6180), 324 (6910), 365 (8400), 390 (10900), 409 (10700). $^1H$ NMR ($D_2O$) $\delta$ 1.43 (3H, d, J=6 Hz), 1.49 (3H, d, J=6 Hz), 1.59 (3H, s), 2.92 (3H, s), 3.52 (3H, s), 5.90 (1H, d, J=8 Hz), 6.15 (1H, d, J=4 Hz).

| Anal. Calcd. for $C_{36}H_{40}N_2O_{14} \cdot 2CF_3COOH \cdot 3.5H_2O$: | | | |
|---|---|---|---|
| | C 47.29, | H 4.86, | N 2.76. |
| Found: | C 47.18, | H 4.22, | N 2.71. |

(b) Synthesis of 6-0-L-Alanylelsamicin A (7e)

93.7 mg (98%), MP 173-175 °C (dec.). IR $\nu_{max}$ (KBr) $cm^{-1}$ 3400, 1780, 1730, 1610, 1370, 1260, 1180. UV $\lambda_{max}$ (MeOH) nm ($\epsilon$) 237 (37400), 268 (34400), 341 (5800), 413 (11100). $^1H$ NMR ($D_2O$) $\delta$ 1.1-1.7 (9H, m), 1.85 (3H, d, J = 7 Hz), 2.8 (3H, s), 3.5 (3H, s), 5.85 (1H, br.), 6.05 (1H, br.).

| Anal. Calcd. for $C_{36}H_{40}N_2O_{14}\bullet 2CF_3COOH\bullet 3.5H_2O$: | | | |
|---|---|---|---|
| | C 47.29, | H 4.86, | N 2.76. |
| Found: | C 47.49, | H 4.26, | N 2.69. |

(c) Synthesis of 6-0-D-Alanylelsamicin A (7f)

91.2 mg (96%), MP 187-189 °C (dec.). IR $\nu_{max}$ (KBr) $cm^{-1}$ 3400, 1780, 1730, 1370, 1205, 1155, 1105. UV $\lambda_{max}$ (MeOH) nm ($\epsilon$) 237 (37100), 268 (35000), 327 (5790), 412 (10900). $^1H$ NMR ($D_2O$) $\delta$ 1.1-1.75 (9H, m), 2.06 (3H, d, J = 7 Hz), 2.9 (3H, s), 3.45 (3H, s), 5.88 (1H, d, J = 8 Hz), 6.17 (1H, d, J = 4 Hz).

| Anal. Calcd. for $C_{36}H_{40}N_2O_{14}\bullet 3CF_3COOH$: | | | |
|---|---|---|---|
| | C 47.29, | H 4.06, | N 2.63. |
| Found: | C 47.55, | H 4.12, | N 2.62. |

EXAMPLE 11

Synthesis of 6-0-Acetyl-3',4'-0-isopropylideneelsamicin A (8a)

A solution of intermediate 6a (239 mg) and p-toluenesulfonic acid monohydrate (150 mg) in acetone (0.5 ml) was kept at room temperature for 10 minutes and evaporated. The residue was dissolved in $CHCl_3$ (20 ml) and the solution was washed with water and brine. The organic solution was dried over $Na_2SO_4$ and evaporated in vacuo. The residue was purified by column chromatography on silica gel (15x120 mm) using MeOH in $CHCl_3$ as an eluant to give 89 mg (42%) of the title compound.

MP 220-230 °C (dec.). IR $\nu_{max}$ (KBr) $cm^{-1}$ 1740, 1500, 1370, 1255, 1215, 1130, 1110, 1070, 1050, 780. UV $\lambda_{max}$ (MeOH) nm ($\epsilon$) 237 (33500), 267 (36800), 309 (5420), 324 (6560), 363 (7960), 388 (10200), 408 (10500), 449 (1250). $^1H$ NMR ($CDCl_3$) $\delta$ 1.3-1.5 (12H, m), 1.73 (3H, s), 2.48 (3H, s), 2.80 (3H, s), 3.34 (3H, s), 5.46 (1H, d, J = 8 Hz), 5.88 (1H, d, J = 4 Hz).

EXAMPLE 12

Synthesis of 6-0-n-Hexanoyl-3',4'-0-isopropylidene-elsamicin A (8b)

A mixture of intermediate 6b (60 mg) and 99% trifluoroacetic acid (0.5 ml) was kept at room temperature. After 5 minutes, the mixture was evaporated under reduced pressure. The residue was triturated with isopropyl ether to give yellow powder, which was purified by silica gel column chromatography using MeOH in $CHCl_3$ as an eluant to give 32 mg (66%) of the desired product.

MP 160-164 °C. IR $\nu_{max}$ (KBr) $cm^{-1}$ 1730, 1355, 1240, 1115, 1060, 765. UV $\lambda_{max}$ (MeOH) nm ($\epsilon$) 236 (36200), 267 (39500), 309 (6140), 324 (7440), 364 (8760), 388 (11300), 408 (11600). $^1H$ NMR ($CDCl_3$) $\delta$ 0.97 (3H, t, J = 7 Hz), 1.30 (3H, d, J = 6 Hz), 1.37 (3H, d, J = 6 Hz), 1.42 (3H, s), 1.46 (3H, s), 1.70 (3H, s), 2.87 (3H, s), 3.37 (3H, s), 5.25 (1H, d, J = 8 Hz), 5.80 (1H, d, J = 4 Hz).

MS (SIMS): M/Z 792 $(M+H)^+$, 694, 433, 360, 334, 160, 99, 86.

EXAMPLE 13

Synthesis of 3',4'-0-Isopropylidene-6-0-(N-trifluoroacetyl-$\beta$-alanyl)elsamicin A (8c)

A solution of intermediate 6c (127 mg) and TsOH (77 mg) in acetone (1.5 ml) was kept at room temperature for 15 minutes. The reaction mixture was evaporated to remove the solvent. The residue was dissolved again in acetone and kept at room temperature for 15 minutes. Evaporation and dissolution were

repeated until the starting material disappeared on the TLC ($CHCl_3$/MeOH = 9:1). The residue was dissolved in acetone (1.5 ml) and 2,2-dimethoxypropane (0.5 ml) was added to the solution. The reaction mixture was stood at ambient temperature for 1 hour and evaporated in vacuo to give an oily residue, which was dissolved in $CHCl_3$ (10 ml) and the solution was washed twice with ice water, dried over $MgSO_4$ and evaporated under reduced pressure to give a yellow mass, which was triturated with ether to give 110 mg (81%) of compound 8c as yellow powder.

MP 186-187°C. IR $\nu_{max}$ (KBr) $cm^{-1}$ 1740, 1720, 1500, 1370, 1255, 1215, 1170, 1120, 1070. UV $\lambda_{max}$ - (MeOH) nm ($\epsilon$) 229 (35500), 236 (37300), 267 (12500), 309 (6680), 323 (8020), 364 (9260), 388 (11800), 427 (11700). $^1$H NMR ($CDCl_3$ + $CD_3OD$) $\delta$ 1.18 (3H, d, J=6 Hz), 1.40 (3H, s), 1.43 (3H, d, J=6 Hz), 1.49 (3H, s), 1.64 (3H, s), 2.38 (3H, s), 3.47 (3H, s), 5.73 (1H, d, J=8 Hz), 5.96 (1H, d, J=4 Hz), 7.10 (2H, d, J=8 Hz), 7.58 (2H, d, J=8 Hz).

| Anal. Calcd. for $C_{41}H_{43}N_2O_{15}F_3$•TsOH•3/2$H_2O$: | | | |
|---|---|---|---|
| | C 54.39, | H 5.13, | N 2.64. |
| Found: | C 54.37, | H 4.94, | N 2.54. |

EXAMPLE 14

Synthesis of 6-0-$\beta$-Alanyl-3',4'-0-isopropylidene-elsamicin A (8d)

A solution of intermediate 6d (96.5 mg) and p-TsOH (85 mg) in dry acetone (2 ml) was stirred at room temperature for 2.5 hours and evaporated in vacuo. The residue was dissolved in a mixture of 10% MeOH in $CHCl_3$ and the solution was washed with water and brine, dried over $MgSO_4$ and evaporated in vacuo. The residue was triturated with ether to give 44 mg (47%) of compound 8d.

MP 191-193°C (dec.). IR $\nu_{max}$ (KBr) $cm^{-1}$ 3380, 1730. UV $\lambda_{max}$ (MeOH) nm ($\epsilon$) 236 (29600), 267 (32800), 309 (5080), 324 (6180), 364 (7170), 388 (9150), 407 (9130). $^1$H NMR ($CDCl_3$ + $CD_3OD$ + $D_2O$) $\delta$ 1.1-1.6 (15H, m), 2.32 (3H, s), 2.85 (3H, s), 3.07 (2H, m), 3.45 (3H, s), 5.56 (1H, d, J=8 Hz), 5.96 (1H, d, J=4 Hz), 7.02 (2H, d, J=8 Hz), 7.64 (2H, d, J=8 Hz).

EXAMPLE 15

Synthesis of 6-0-L-Alanyl-3',4'-0-isopropylidene-elsamicin A (8e)

A solution of intermediate 6e (145 mg) and p-TsOH (143 mg) in dry acetone (3 ml) was stirred at room temperature for 2 hours and evaporated in vacuo. The residue was dissolved in $CHCl_3$ (20 ml) and the solution was washed with water and brine, dried over $MgSO_4$ and evaporated in vacuo in the presence of a small amount of $CH_3COOH$. The residue was triturated with ether and the precipitates were collected by filtration to give 71.7 mg (51%) of compound 8e.

MP 208-210°C (dec.). IR $\nu_{max}$(KBr) $cm^{-1}$ 3400, 1735, 1495, 1370, 1215, 1125, 1070. UV $\lambda_{max}$ (MeOH) nm ($\epsilon$) 237 (36700), 267 (39200), 309 (6150), 324 (7400), 363 (8740), 388 (11200), 407 (11300). $^1$H NMR ($CDCl_3$ $\delta$ 1.45 (21H, m), 2.68 (3H, s), 3.29 (3H, s), 5.45 (1H, br.), 5.85 (1H, br.).

| Anal. Calcd. for $C_{39}H_{44}N_2O_{14}$•1/2TsOH•5/2AcOH•3/2H2O: | | | | |
|---|---|---|---|---|
| | C 55.49, | H 5.98, | N 2.72, | S 1.52. |
| Found: | C 55.55, | H 5.64, | N 2.73, | S 1.62. |

EXAMPLE 16

Synthesis of 6-0-D-Alanyl-3',4'-0-isopropylidene-elsamicin A (8f)

A solution of intermediate 6f (145 mg) and p-TsOH (143 mg) in dry acetone (3 ml) was stirred at room temperature for 2 hours and evaporated in vacuo. The residue was dissolved in $CHCl_3$ (20 ml) and the solution was washed with water and brine, dried over $MgSO_4$ and evaporated in vacuo in the presence of a

small amount of $CH_3COOH$. The residue was triturated with ether and the precipitates were collected by filtration to give 95.6 mg (68%) of compound 8f.

MP 217-219 °C (dec.). IR $\nu_{max}$ (KBr) $cm^{-1}$ 3400, 1730, 1495, 1370, 1215, 1070, 1025. UV $\lambda_{max}$ (MeOH) nm ($\epsilon$) 236 (32900), 267 (36400), 309 (5730), 324 (6900), 363 (8610), 388 (10300), 407 (10300). $^1H$ NMR ($CDCl_3$) $\delta$ 1.45 (21H, m), 2.67 (3H, s), 3.4 (3H, s), 5.24 (1H, d, J = 8 Hz), 5.91 (1H, br.).

| Anal. Calcd. for $C_{39}H_{44}N_2O_{14}$ • 1/2TsOH • 5/2AcOH • 2$H_2O$: | | | | |
|---|---|---|---|---|
| | C 55.01, | H 6.02, | N 2.70, | S 1.54. |
| Found: | C 54.90, | H 5.81, | N 2.60, | S 1.43. |

## Claims

**1.** A compound having the formula:

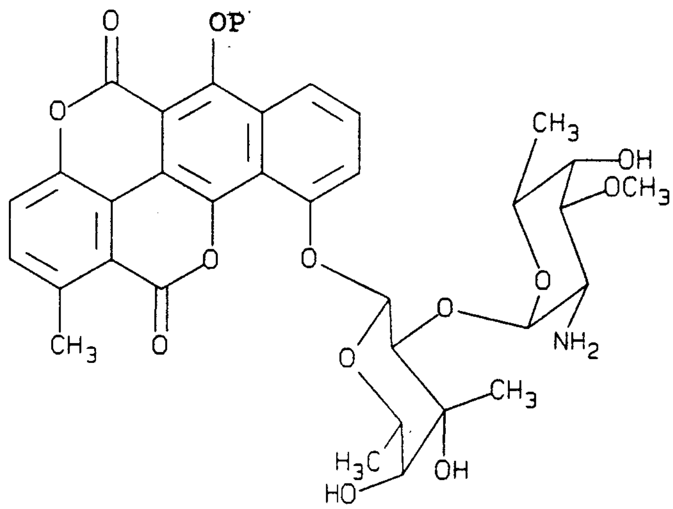

wherein P is COR; and R is $C_1$-$C_5$ alkyl which may be unsubstituted or substituted with amino or acylamino.

**2.** A compound having the formula

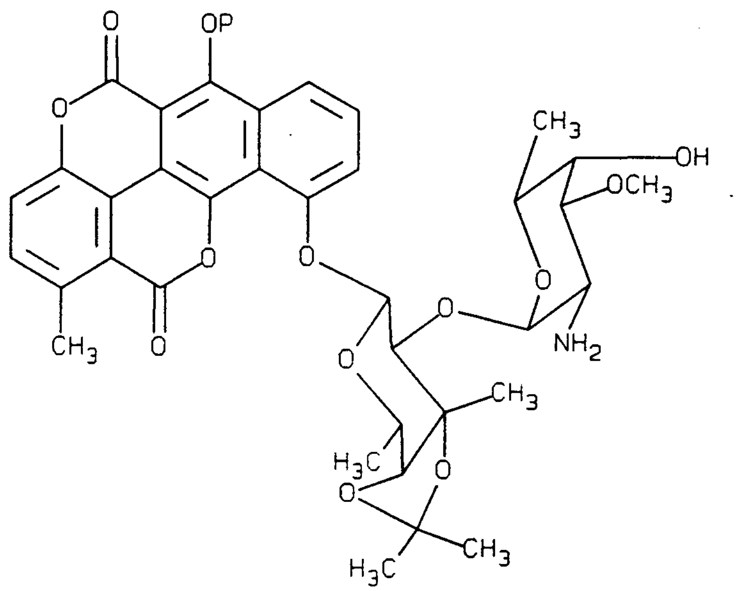

wherein P is COR; and R $C_1$-$C_5$ alkyl which may be unsubstituted or substituted with amino or acylamino.

3. A process for producing a compound as claimed in any one of claims 1 and 6 which comprises O-acylation of N-t-butoxycarbonyl elsamicin A with appropriate carboxylic acids and dicyclohexylcarbodiimide in the presence of a base and subsequent deprotection.

4. A process for producing a compound as claimed in any one of claims 1 and 6 which comprises O-acylation of N-t-butoxycarbonyl-3',4'-O-isopropylideneelsamicin A with appropriate carboxylic acids and subsequent deprotection,preferably with trifluoroacetic acid.

5. A process for producing a compound as claimed in any one of claims 2 and 7 which comprises acylation of N-t-butoxycarbonyl-3',4'-0-isopropylidene elsamicin A with appropriate carboxylic acids and a dehydrating agent, preferably dicyclohexylcarbodiimide in the presence of a base, and subsequent deprotection, preferably with p-toluenesulfonic acid in acetone or trifluoroacetic acid.

6. The compound of Claim 1 which is
 6-O-acetylelsamicin A;
 6-O-n-hexanoylelsamicin A;
 6-O-(N-trifluoro-acetyl-β-alanyl)elsamicin A;
 6-O-β-alanylelsamicin A;
 6-O-L-alanylelsamicin A; or
 6-O-D-alanylelsamicin A.

7. The compound of Claim 2 which is
 6-O-acetyl-3',4'-O-isopropylideneelsamicin A;
 6-O-n-hexanoyl-3',4'-O-isopropylideneelsamicin A;
 3',4'-O-isopropyl-idene-6-O-(N-trifluoroacetyl-β-alanyl)elsamicin A;
 6-O-β-alanyl-3',4'-O-isopropylideneelsamicin A;
 6-O-L-alanyl-3',4'-O-isopropylideneelsamicin A; or
 6-O-D-alanyl-3',4'-O-isopropylideneelsamicin A.

8. 2''-N-t-butoxycarbonylelsamicin A.

9. 2''-N-t-butoxycarbonyl-3',4'-O-isopropylideneelsamicin A.

10. 6-O-acetyl-2''-N-t-butoxy-carbonyl-3',4'-O-isopropylideneelsamicin A.

**11.** 2''-N-t-butoxycarbonyl-6-O-n-hexanoyl-3',4'-O-isopropylideneelsamicin A.

**12.** 2''-N-t-butoxycarbonyl-3',4'-O-isopropylidene-6-O-(N-trifluoroacetyl-β-alanyl)-elsamicin A.

**13.** 2''-N-t-butoxycarbonyl-6-O-(N-t-butoxycarbonyl-β-alanyl)-3',4'-O-isopropylidene-elsamicin A.

**14.** 2''-N-t-butoxycarbonyl-6-O-(N-t-butoxycarbonyl-L-alanyl)-3',4'-O-isopropylidene-elsamicin A.

**15.** 2''-N-t-butoxycarbonyl-6-O-(N-t-butoxycarbonyl-D-alanyl)-3',4'-O-isopropylidene-elsamicin A.

**16.** A pharmaceutical formulation which comprises as an active ingredient a compound as claimed in any one of claims 1, 2, 6-15, associated with one or more pharmaceutical acceptable carriers or diluents.

**17.** The use of a compound as claimed in any one of claims 1, 2, 6-15 for preparing a pharmaceutical composition for treating tumors.

**Claims for the following Contracting State : ES**

**1.** A process for preparing the compound having the formula:

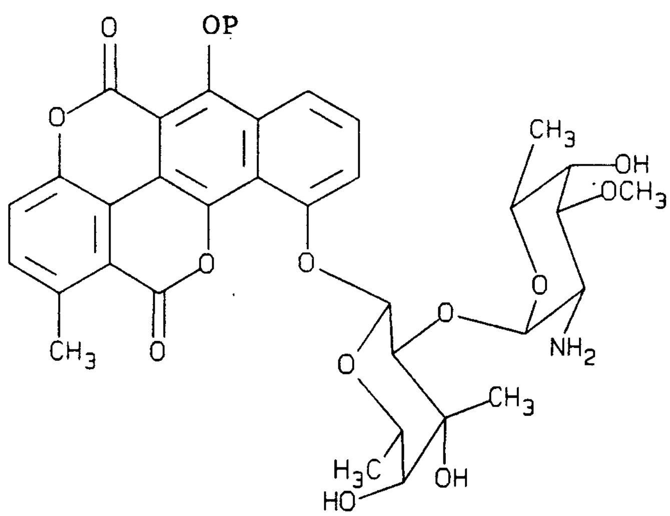

wherein P is COR; and R is $C_1$-$C_5$ alkyl which may be unsubstituted or substituted with amino or acylamino, which comprises O-acylation of N-t-butoxycarbonyl elsamicin A with appropriate carboxylic acids and a dehydrating agent, preferably dicyclohexylcarbodiimide in the presence of a base, and subsequent deprotection.

**2.** A process for preparing the compound having the formula:

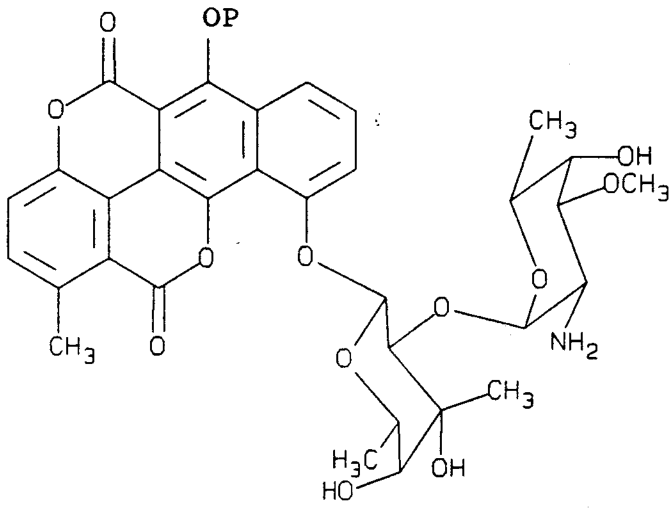

wherein P is COR; and R is $C_1$-$C_5$ alkyl which may be unsubstituted or substituted with amino or acylamino, which comprises O-acylation of N-t-butoxycarbonyl-3',4'-O-isopropylideneelsamicin A with appropriate carboxylic acids and subsequent deprotection, preferably with trifluoroacetic acid.

**3.** A process for preparing the compound having the formula

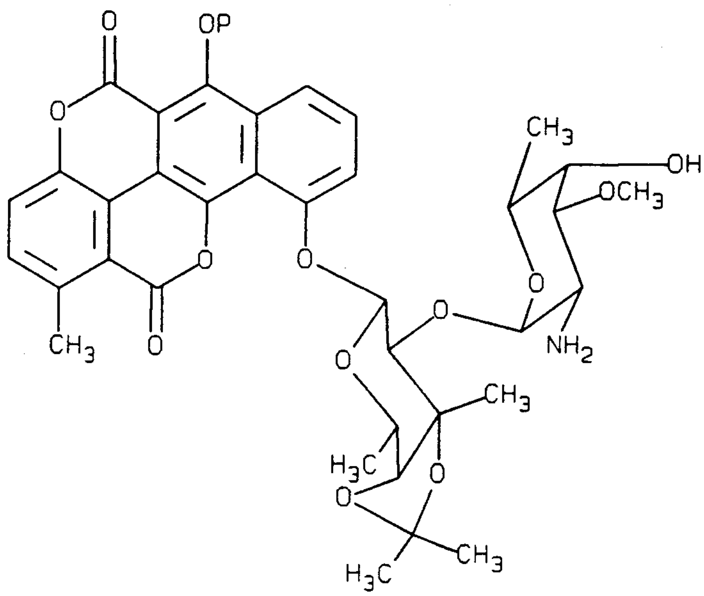

wherein P is COR; and R $C_1$-$C_5$ alkyl which may be unsubstituted or substituted with amino or acylamino, which comprises acylation of N-t-butoxycarbonyl-3',4'-0-isopropylidene elsamicin A with appropriate carboxylic acids and a dehydrating agent, preferably dicyclohexylcarbodiimide in the presence of a base, and subsequent deprotection, preferably with p-toluenesulfonic acid in acetone or trifluoroacetic acid.

**4.** A process as claimed in any one of claims 1 or 2 for preparing
6-O-acetyl-elsamicin A;
6-O-n-hexanoyl-elsamicin A;
6-O-(N-trifluoro-acetyl-$\beta$-alanyl)elsamicin A;
6-O-$\beta$-alanyl-elsamicin A;
6-O-L-alanyl-elsamicin A; or
6-O-D-alanyl-elsamicin A.

5. A process as claimed in claim 3 for preparing
   6-O-acetyl-3',4'-O-isopropylideneelsamicin A;
   6-O-n-hexanoyl-3',4'-O-isopropylideneelsamicin A;
   3',4'-O-isopropyl-idene-6-O-(N-trifluoroacetyl-β-alanyl)elsamicin A;
   6-O-β-alanyl-3',4'-O-isopropylideneelsamicin A;
   6-O-L-alanyl-3',4'-O-isopropylideneelsamicin A; or
   6-O-D-alanyl-3',4'-O-isopropylideneelsamicin A.

6. The process for preparing the intermediate 2''-N-t-butoxycarbonylelsamicin A which comprises reacting elsamicin A, di-t-butyl dicarbonate in the pesence of a suitable base, preferably triethylamine.

7. The process for preparing the intermediate 2''-N-t-butoxycarbonyl-3',4'-O-isopropylideneelsamicin A which comprises 2''-N-t-butoxycarbonylelsamicin A with 2,2-dimethoxypropane in the presence of an acidic catylst, preferably p-toluene sulfonic acid.

8. The process for preparing the intermediate 6-O-acetyl-2''-N-t-butoxycarbonyl-3',4'-O-isopropyl ideneelsamicin A, which comprises acetylating 2''-N-t-butoxycarbonyl-3',4'-O-isopropylideneelsamicin A, preferably with acetic acid and dicyclohexylcarbodiimide in the presence of a base.

9. The process for preparing the intermediate 2''-N-t-butoxycarbonyl-6-O-n-hexanoyl-3',4'-O-isopropylideneelsamicin A which comprises hexanoylating 2''-N-t-butoxycarbonyl-3',4'-O-isopropylideneelsamicin A, preferably with caproic acid and dicyclohexylcarbodiimide in the presence of a base.

10. The process for preparing the intermediate 2''-N-t-butoxycarbonyl-3',4'-O-isopropylidene-6-O-(N-trifluoroacetyl-β-alanyl)elsamicin A, which comprises reacting 2''-N-t-butoxycarbonyl-3',4'-O-isopropylideneelsamicin A with a reagent for introducing N-trifluoroacetyl-β-alanyl radical, preferably with N-trifluoroacetyl-β-alanine, dicyclohexyl-carbodiimide and a base.

11. The process for preparing the intermediate 2''-N-t-butoxycarbonyl-6-O-(N-t-butoxycarbonyl-β-alanyl)-3',4'-O-isopropylideneelsamicin A, which comprises reacting 2''-N-t-butoxycarbonyl-3',4'-O-isopropylideneelsamicin A with a reagent for introducing the N-t-butoxycarbonyl-β-alanyl radical, preferably with N-t-butoxycarbonyl-β-alanine, dicyclohexyl-carbodiimide and a base.

12. The process for preparing the intermediate 2''-N-t-butoxycarbonyl-6-O-(N-t-butoxycarbonyl-L-alanyl)-3',4'-O-isopropylideneelsamicin A, which comprises reacting 2''-N-t-butoxycarbonyl-3',4'-O-isopropylideneelsamicin A with a reagent for introducing the N-t-butoxycarbonyl-L-alanyl radical, preferably with N-t-butoxycarbonyl-L-alanine, dicyclohexyl-carbodiimide and a base.

13. The process for preparing the intermediate 2''-N-t-butoxycarbonyl-6-O-(N-t-butoxycarbonyl-D-alanyl)-3',4'-O-isopropylideneelsamicin A, which comprises reacting 2''-N-t-butoxycarbonyl-3',4'-O-isopropylideneelsamicin A with a reagent for introducing the N-t-butoxycarbonyl-D-alanyl radical, preferably with N-t-butoxy-D-alanine, dicyclohexyl-carbodiimide and a base.

14. A process for preparing a pharmaceutical composition which comprises admixing a compound as defined in any one of claims 1 - 13 with one or more pharmaceutical carriers, or diluents.

15. The use of a compound of any one of claims 1 - 13 for preparing a pharmaceutical composition for treating or preventing tumors.

**Claims for the following Contracting State : GR**

1. A compound having the formula:

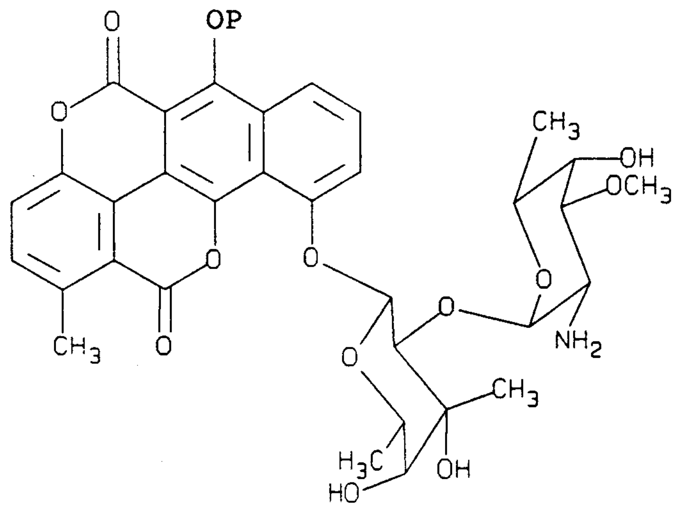

wherein P is COR; and R is $C_1$-$C_5$ alkyl which may be unsubstituted or substituted with amino or acylamino.

**2.** A compound having the formula

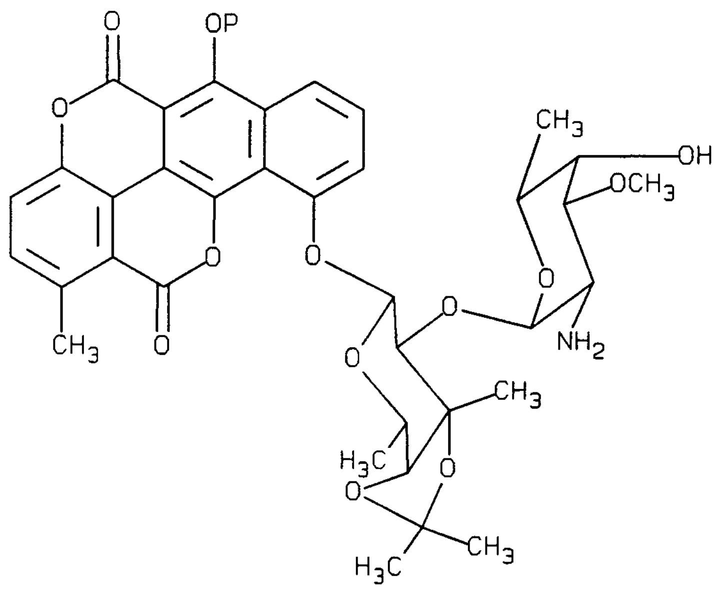

wherein P is COR; and R $C_1$-$C_5$ alkyl which may be unsubstituted or substituted with amino or acylamino.

**3.** A process for producing a compound as claimed in any one of claims 1 and 6 which comprises O-acylation of N-t-butoxycarbonyl elsamicin A with appropriate carboxylic acids and dicyclohexylcarbodiimide in the presence of a base and subsequent deprotection.

**4.** A process for producing a compound as claimed in any one of claims 1 and 6 which comprises O-acylation of N-t-butoxycarbonyl-3',4'-O-isopropylideneelsamicin A with appropriate carboxylic acids and subsequent deprotection, preferably with trifluoroacetic acid.

**5.** A process for producing the producing a compound as claimed in any one of claims 2 and 7 which comprises acylation of N-t-butoxycarbonyl-3',4'-0-isopropylidene elsamicin A with appropriate carboxylic acids and a dehydrating agent, preferably dicyclohexylcarbodiimide in the presence of a base, and

subsequent deprotection, preferably with p-toluenesulfonic acid in acetone or trifluoroacetic acid.

**6.** The compound of Claim 1 which is
6-O-acetylelsamicin A;
6-O-n-hexanoylelsamicin A;
6-O-(N-trifluoro-acetyl-$\beta$-alanyl)elsamicin A;
6-O-$\beta$-alanylelsamicin A;
6-O-L-alanylelsamicin A; or
6-O-D-alanylelsamicin A.

**7.** The compound of Claim 2 which is
6-O-acetyl-3',4'-O-isopropylideneelsamicin A;
6-O-n-hexanoyl-3',4'-O-isopropylideneelsamicin A;
3',4'-O-isopropyl-idene-6-O-(N-trifluoroacetyl-$\beta$-alanyl)elsamicin A;
6-O-$\beta$-alanyl-3',4'-O-isopropylideneelsamicin A;
6-O-L-alanyl-3',4'-O-isopropylideneelsamicin A; or
6-O-D-alanyl-3',4'-O-isopropylideneelsamicin A.

**8.** 2''-N-t-butoxycarbonylelsamicin A.

**9.** 2''-N-t-butoxycarbonyl-3',4'-O-isopropylideneelsamicin A.

**10.** 6-O-acetyl-2''-N-t-butoxy-carbonyl-3',4'-O-isopropylideneelsamicin A.

**11.** 2''-N-t-butoxycarbonyl-6-O-n-hexanoyl-3',4'-O-isopropylideneelsamicin A.

**12.** 2''-N-t-butoxycarbonyl-3',4'-O-isopropylidene-6-O-(N-trifluoroacetyl-$\beta$-alanyl)-elsamicin A.

**13.** 2''-N-t-butoxycarbonyl-6-O-(N-t-butoxycarbonyl-$\beta$-alanyl)-3',4'-O-isopropylidene-elsamicin A.

**14.** 2''-N-t-butoxycarbonyl-6-O-(N-t-butoxycarbonyl-L-alanyl)-3',4'-O-isopropylidene-elsamicin A.

**15.** 2''-N-t-butoxycarbonyl-6-O-(N-t-butoxycarbonyl-D-alanyl)-3',4'-O-isopropylidene-elsamicin A.

**16.** A process for preparing a pharmaceutical formulation which comprises admixing a compound as claimed in any one of claims 1, 2, 6-15 with one or more pharmaceutical acceptable carriers or diluents.

**17.** The use of a compound as claimed in any one of claims 1, 2, 6-15 for preparing a pharmaceutical composition for treating tumors.

European Patent Office

# EUROPEAN SEARCH REPORT

Application Number

EP 92 10 9109

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X<br>Y | EP-A-0 219 852 (ISHIHARA SANGYO KAISHA LTD.)<br>* the whole document *<br>--- | 2,7<br>1-17 | C07H17/04<br>A61K31/70 |
| D,Y | US-A-4 518 589 (M.KONISHI ET AL.)<br>* claims 1-8 *<br>--- | 1-17 | |
| Y | EP-A-0 159 708 (ISHIHARA SANGYO KAISHA LTD.)<br>* abstract *<br>--- | 1-17 | |
| Y | EP-A-0 381 114 (ISHIHARA SANGYO KAISHA LTD.)<br>* the whole document *<br>----- | 1-17 | |

TECHNICAL FIELDS SEARCHED (Int. Cl.5 )

C07H
A61K

The present search report has been drawn up for all claims

| Place of search | Date of completion of the search | Examiner |
|---|---|---|
| THE HAGUE | 29 JULY 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)